# EUROPEAN PATENT APPLICATION

(11) **EP 4 631 372 A1**
(43) Date of publication of application: **15.10.2025**
(21) Application number: 25169432.9
(22) Date of filing: 09.04.2025
(51) Int. Cl.: A24F 40/40, A24F 40/60, G09F 9/33

(54) **ELECTRONIC VAPING DEVICE**

(30) Priority: 09.04.2024 CN 202410424436
(71) Applicant: Shenzhen Geekvape Technology Co., Ltd., Shenzhen, Guangdong 518100 (CN)
(72) Inventor: ZHOU, Lin, Shenzhen, 518100 (CN); LI, Mushan, Shenzhen, 518100 (CN); CHEN, Qiang, Shenzhen, 518100 (CN)
(74) Representative: Manitz Finsterwald Patent- und Rechtsanwaltspartnerschaft mbB

(57) **Abstract**

Disclosed is an electronic vaping device which comprising: a housing (1); a mouthpiece (151); a vaping component; a flexible display panel (2); a control component; the housing (1) comprises a top surface, a bottom surface, and a plurality of side surfaces, the mouthpiece (151) is configured on the top surface, areas included in the plurality of side surfaces comprise gripping area and non-gripping area; the gripping area is designed for gripping the housing (1), and the distribution position of the gripping area has a preset positional relationship with the preset position of the mouthpiece (151); the flexible display panel (2) is configured on one or more selected from the group consisting of the non-gripping area, the top surface, and the bottom surface; and the flexible display panel (2) is distributed on at least two selected from the group consisting of the top surface, bottom surface, and the plurality of side surfaces.

## Description

### TECHNICAL FIELD

This invention relates to the field of vaping technology, and specifically, the invention also relates to an electronic vaping device.

### BACKGROUND

With the development of electronic technology, diverse electronic vaping products have appeared on the market, such as electronic vaping devices. The electronic vaping device may comprise a built-in power supply, vaping component, vaping substrate, etc., while it is working, energy is provided by the built - in power supply, and aerosol is generated by heating the vaping substrate to vape.

With the usage of the electronic vaping device, the built-in power supply and the amount of vaping substrate in the electronic vaping device will decrease, rendering it useless. Therefore, users want to acquire the current power or vaping capacity of the electronic vaping device to adjust their usage. Meanwhile, electronic vaping devices also have different working modes, as well as different alarm status and other information. Users also hope to acquire such information to use the electronic vaping device properly. Therefore, electronic vaping devices are equipped with display panels to display various information.

In the present technical solution, a lot of information needs to be displayed, but the display panel can only be configured on one end surface of the electronic vaping device, and its display area is relatively small, which cannot satisfy the display need. Therefore, innovative technologies need to be proposed.

### SUMMARY

The technical problem addressed by this application is that the display area of the display panel is relatively small and fails to meet the display requirements.

According to a first aspect, an electronic vaping device is provided in an embodiment, comprising:
a housing, inside which a reservoir is configured to store vaping substrate and an air outlet channel is formed in the housing;
a mouthpiece configured on the housing and connected with the air outlet channel;
a vaping component, configured in the housing, the vaping component is designed to vape the vaping substrate to generate aerosol which passes through the air outlet channel to the mouthpiece;
a flexible display panel configured on the housing for displaying visual information;
a control component respectively connected to the flexible display panel and the vaping component;
the housing comprises a top surface, a bottom surface, and a plurality of side surfaces that connect the top surface and the bottom surface, the plurality of side surfaces are connected with each other in sequence, the mouthpiece is arranged at a preset position of the top surface, the plurality of side surfaces comprise gripping area and non-gripping area;
the gripping area is designed for gripping the housing, and the distribution position of the gripping area has a preset positional relationship with the preset position of the mouthpiece, so that when at least part of the gripping area is being gripped by a user, the mouthpiece approaches and/or faces the user;
the flexible display panel is configured on one or more selected from the group consisting of the non-gripping area, the top surface, and the bottom surface, the flexible display panel is distributed on at least two selected from the group consisting of the top surface, bottom surface, and the plurality of side surfaces.

According to a second aspect, an electronic vaping device is provided in an embodiment, comprising:
a housing, inside which a reservoir is configured to store vaping substrate and an air outlet channel is formed in the housing;
a vaping component, configured in the housing, the vaping component is designed to vape the vaping substrate to generate aerosol which passes through the air outlet channel to the outside of the housing;
a flexible display panel configured on the housing for displaying visual information;
a control component respectively connected to the flexible display panel and the vaping component;
the housing comprises a plurality of end surfaces that connect with each other in sequence, the plurality of end surfaces comprise gripping area and non-gripping area; the gripping area is designed for gripping the housing, the flexible display panel is configured on the non-gripping area, the flexible display panel is distributed on at least two end surfaces of the plurality of end surfaces.

According to a third aspect, an electronic vaping device is provided in an embodiment, comprising:
a housing, inside which a reservoir is configured to store vaping substrate and an air outlet channel is formed in the housing
a mouthpiece configured on the housing and connected with the air outlet channel;
a vaping component, configured in the housing, the vaping component is designed to vape the vaping substrate to generate aerosol which passes through the air outlet channel to the mouthpiece;
a flexible display panel configured on the housing for displaying visual information;
a control component respectively connected to the flexible display panel and the vaping component;
the housing comprises a top surface, a bottom surface, and a plurality of side surfaces that connect the top surface and the bottom surface, the plurality of side surfaces are connected with each other in sequence, the mouthpiece is arranged at a preset position of the top surface, the flexible display panel is annularly configured on the plurality of side surfaces.

According to the electronic vaping device of the above embodiment, the display panel of the electronic vaping device is configured as a flexible display panel. For its flexibility characteristics, the flexible display panel can be configured on at least two end surfaces of the housing. So that the display area of the flexible display panel on multiple end surfaces is larger than that of the display panel disposed on one end surface, and the flexible display panel can satisfy the user's display needs. Meanwhile, the area included in the end surface of the housing is configured as gripping area and non-gripping area. The gripping area is designed for users to grip, while the non-gripping area can be configured for the flexible display panel, making the flexible display unshielded by user's gripping when it is arranged on the plurality of end surfaces of the housing.

### BRIEF DESCRIPTION OF DRAWINGS

Figure 1 shows a schematic structural diagram of an electronic vaping device according to an embodiment;
Figure 2 shows a schematic structural diagram of an electronic vaping device according to another embodiment;
Figure 3 shows a schematic structural diagram of a flexible display panel according to an embodiment;
Figure 4 shows a schematic structural diagram of a bent flexible display panel according to an embodiment;
Figure 5 shows a schematic structural diagram of the first housing according to an embodiment;
Figure 6 shows a schematic structural diagram of the second housing according to an embodiment.

### DETAILED DESCRIPTION

The present application will be further-detailed described by combining specific embodiments with drawings. Similar elements in different embodiments are associated with similar element numbers. In the following embodiments, many details are described in order to make the present application easily understood. However, person ordinary skilled in the art can readily recognize that some of the features may be omitted in different situations, or may be replaced by other elements, materials, and methods. In some cases, the reason why some implementations related to the present application are not shown or described in the draft is to avoid overwhelming the core part of the present application with excessive descriptions. For those ordinarily skilled in the art, It is not necessary to describe these related implementations in detail, they can fully understand the related implementations based on the descriptions in the draft and the general technical knowledge in the field.

Additionally, the features, implementations, or characteristics described in the draft may be combined in any suitable way to form various embodiments. At the same time, each step or action in the method description can also be sequentially exchanged or adjusted in a way that is obvious to those ordinarily skilled in the art. Therefore, the various sequences in the description and drawings are only for clearly describing a certain embodiment, and do not imply a necessary sequence, unless otherwise stated that a certain sequence must be followed.

The serial numbers assigned to components in this draft, such as "first", "second", etc., are only designed to distinguish the described objects and do not have any sequential or technical meaning. The terms "connection" mentioned in this draft illustrate direct and indirect connections unless otherwise specified.

In present technical solutions, the display panel of the electronic vaping device is usually set on a PCB board, and a digital tube provided on the PCB board to display the status information of the electronic vaping device. Therefore, the electronic vaping device is usually equipped with a display panel on one side of the electronic vaping device. The display panel is configured to display various information such as the remaining amount of vaping substrate, remaining power, and working mode of the electronic vaping device. Since the size of electronic vaping device is relatively small, even if the entire side surface of the electronic vaping device is configure for the display panel, the display area of the display panel is still relatively small, and cannot satisfy the display needs.

In some embodiments of the present application, the display panel of the electronic vaping device is configured as a flexible display panel, and the flexible display panel can be configured on at least two end surfaces of the housing for its flexibility characteristics, so that the display area of the flexible display panel is distributed on multiple end surfaces of the housing, compared with the display panel being disposed on only one end surface, the display area of the flexible display panel can satisfy the user's display needs better for it enlarged the display area of the flexible display panel. At the same time, since the flexible display panels are disposed on multiple end surfaces of the housing, the user may shield the flexible display panel when gripping the housing, thereby affecting the display performance of the flexible display panel, Regarding this, the application also sets the area included in the end surface of the housing as a griping area and a non-gripping area, where the griping area is designed for the user to grip, while the non-gripping area can be configure for the flexible display panel. Therefore, even if the flexible display panel is arranged on multiple end surfaces of the housing, it will not be shield by the user's holding.

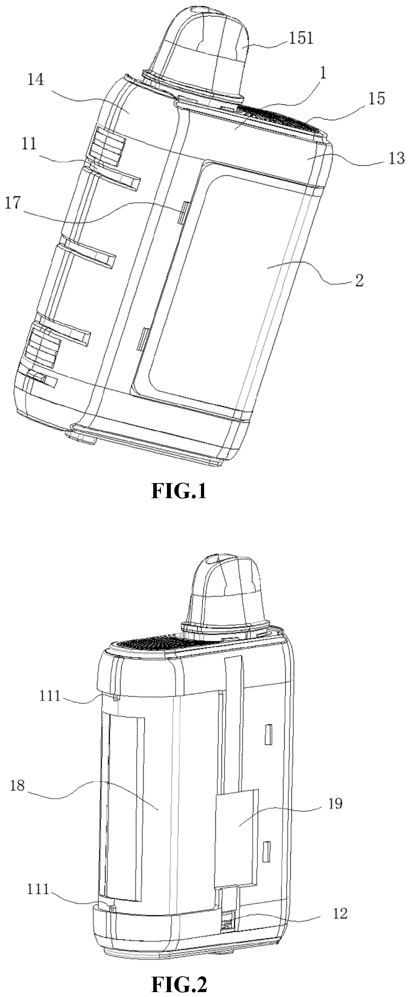

Some embodiments provide an electronic vaping device that is designed to vape a stored vaping substrate to generate outputting aerosol, wherein the vaping substrate can be e-liquid, liquid medicine, etc. Refer to Figure 1, the electronic vaping device comprises a housing 1, a mouthpiece 151, a vaping component, a flexible display panel 2 and a control component. The following provides specific explanations for them.

The housing 1 is designed for the installation and fixation of other components and for storing the vaping substrate. In some embodiments, a reservoir configured to store vaping substrate is formed inside the housing 1. For example, the reservoir can be designed to store the vaping substrate, or the reservoir can also be designed to install a storage element, and the storage element is designed to store the vaping substrate. The storage element may be a porous material, such as liquid storage cotton, and the vaping substrate may be e-liquid, liquid medicine, etc. In some embodiments, inside the housing 1 may form an air inlet channel, a vaping channel, and an air outlet channel that are connected to the reservoir. Wherein, the air inlet channel and the air outlet channel can be respectively formed at opposite ends of the housing 1, and the reservoir can be designed around the outer periphery of the vaping channel.

The mouthpiece 151 is configured on the housing 1 and communicates with the air outlet channel of the housing 1.

The vaping component is installed to the housing 1, for vaping the vaping substrate to generate aerosol, and outputs the aerosol to the mouthpiece 151 through the air outlet channel of the housing 1, and then outputs the aerosol to the outside of the housing 1. In some embodiments, the vaping component can be disposed in the vaping channel to vape the vaping substrate to generate an aerosol. In some embodiments, the vaping component can vape the vaping substrate to generate an aerosol by heating. In some embodiments, the vaping substrate in the reservoir can penetrate into the vaping component, so that the vaping component can vape the vaping substrate into an aerosol, the airflow enters the electronic vaping device through the air inlet channel of the housing 1, then flows into the vaping channel, and carries the aerosol from the vaping channel and flows out through the air outlet channel.

The flexible display panel 2 is installed on the surface of the housing 1 for displaying visual information. In some embodiments, the housing 1 has a plurality of end surfaces connected in sequence, and the flexible display panel 2 is configured on at least two of the plurality of end surfaces, thereby increasing the display area for the flexible display panel 2, the flexible display panel 2 is designed to display status information of the electronic vaping device. In this embodiment, since the flexible display panel 2 is flexible, it can be bent to a certain extent. Therefore, after the flexible display panel 2 is bent, it can be fixed to multiple end surfaces of the housing 1, for example, the flexible display panel 2 may fixed to any two end surfaces on the housing 1, or three end surfaces on the housing 1, or four end surfaces bent in annular shape on the housing 1. In some embodiments, at least one of the multiple end surfaces of the housing 1 can be configured as a curved surface, thereby facilitating the flexible display panel 2 to be fixed to the multiple end surfaces.

Refer to Figure 1 again, in some embodiments, the housing 1 can be composed of multiple housings assembled together, and the housing 1 can also be an integrated structure. In some embodiments, the housing 1 may comprise a plurality of side surfaces connected in sequence, such as a first side surface 11, a second side surface (not shown), a third side surface 13, and a fourth side surface 14. The first side surface 11, the second side surface, the third side surface 13 and the fourth side surface 14 are linked together in order to form an annular surface. An arc transition structure is arranged at the joint of the annular surface which formed by the first side surface 11, the second side surface, the third side surface 13 and the fourth side surface 14, so that the shape of the housing 1 is more rounded, which is convenient for the user to grip and improves the usage experience. The housing 1 also comprises a top surface 15 and a bottom surface (not shown). Both the top surface 15 and the bottom surface are connected to the first side surface 11, the second side surface, the third side surface 13 and the fourth side surface 14. In some embodiments, the mouthpiece 151 is designed at a preset position on the top surface 15, and the user can inhale the aerosol through the mouthpiece 151, the air inlet can be disposed on the bottom surface.

In some embodiments, the housing 1 may designed as a flat square structure, the first side surface 11 and the third side surface 13 are relatively parallel, the second side surface and the fourth side surface 14 are relatively parallel, and size of the first side surface 11 and the third side surface 13 are larger than the size of the second side surface and the fourth side surface 14. For example, the widths of the second side surface and the fourth side surface are less than The widths of the first side surface and the third side surface. In some embodiments, the housing 1 can also be some other shapes, such as a cylinder or an ellipse, and the circumferential side surface of the housing 1 corresponds to a smoothly transitioning annular surface.

The control component is connected to the flexible display panel 2 and the vaping component respectively, and the control component is configured to control the flexible display panel 2 to display the device status of the electronic vaping device, and the control component is also configured to control the vaping component to operate with preset parameters, thereby vape the vaping substrate. In some embodiments, the control component can be implemented based on devices with control functions such as microcontrollers, processors, and controllers.

In some embodiments, the areas included in the multiple end surfaces of the housing 1 can be divided into gripping area and non-gripping area. Wherein, the gripping area is designed for users to grip the housing 1 or the electronic vaping device, while the flexible display panel 2 is arranged in the non-gripping area of the housing 1, and the flexible display panel 2 is distributed on at least two end surfaces of the plurality of end surfaces, so that users can avoid shielding the flexible display panel 2 when gripping the housing 1 or the electronic vaping device. In some embodiments, the mouthpiece 151 is designed at a preset position on one end surface of the housing 1, and the mouthpiece 151 is connected to the air outlet channel, and the preset position of the mouthpiece 151 and the distribution position of the gripping area are preset related, makes the gripping area closer to the mouthpiece 151 than the non-gripping area, or makes the gripping area farther away from the mouthpiece 151 than the non-gripping area. In some embodiments, the preset position is a position offset from the center of one of the end surfaces.

In some embodiments, the area included in the multiple side surfaces of the housing 1 can also be divided into a gripping area and a non-gripping area, and the preset position of the top surface 15 of the housing 1 is configure for the mouthpiece 151. There is a preset positional relationship between the distribution position of the gripping area and the preset position of the mouthpiece 151, so that after at least part of the gripping area is gripped by the user, the mouthpiece 151 approaches and/or faces the user. In this embodiment, since the user is gripping the housing 1 or the electronic vaping device, usually to inhale the aerosol based on the mouthpiece 151, therefore, based on the position of the mouthpiece 151, optimal gripping areas can be designed on multiple end surfaces or multiple side surfaces.

The user can use the mouthpiece 151 to inhale the aerosol in a better way when gripping the optimal gripping areas of the housing 1, for example, by making the mouthpiece 151 close to and/or toward the user.

In some embodiments, the gripping area is an area included in the second side surface of the housing 1, and at least part of the area included in the first side surface that is connected to the second side surface, and/or at least part of the area included in the third side surface that is connected to the second side surface. Other areas of the other side surfaces of the housing 1 are non-gripping areas.

In some embodiments, the gripping area is an area included in the fourth side surface of the housing 1, and at least part of the area included in the first side surface that is connected to the fourth side surface, and/or at least part of the area included in the third side surface that is connected to the fourth side surface. Other areas of the other side surfaces of the housing 1 are non-gripping areas.

In the above embodiment, since the widths of the second side surface and the fourth side surface are smaller than the widths of the first side surface and the third side surface, when the user grips the housing 1, the best gripping area for the user's fingers are the first side surface and the third side surface. So at least part of the first side surface and the third side surface is the gripping area, and the user's palm will cover the second side surface or the fourth side surface, so the second side surface or the fourth side surface are also gripping areas. Meanwhile, At least part of the gripping area on the first side surface and the third side surface can be a small part area of them and away from the mouthpiece 151, it can also be a large part area of them and close to the mouthpiece 151. The gripping area is closer to the mouthpiece 151 than the non-gripping area, or the gripping area is further away from the mouthpiece 151 than the non-gripping area, so as to satisfy the gripping habits and usage habits of the mouthpiece 151 of different users.

In some embodiments, the preset position of the mouthpiece 151 is a position offset from the center of the end surface where the mouthpiece 151 is located. For example, when the preset position of the top surface 15 of the housing 1 is designed to configure the mouthpiece 151, then the preset position is a position close to the second side surface or the fourth side surface. So that it is easy to divide the gripping area close to the mouthpiece 151, and the gripping away from the mouthpiece 151.

In the above embodiment, the area included in the housing 1 is divided into a gripping area and a non-gripping area, and the flexible display panel 2 is arranged in the non-gripping area, so that while increasing the display area of the flexible display panel 2, it can prevent the user from shielding the flexible display panel 2 by gripping it. Furthermore, the gripping area and the non-gripping area can also be configured according to the position of the mouthpiece 151, so that the user can inhale the aerosol through the mouthpiece 151more conveniently based on the gripping area.

The above are some descriptions of the electronic vaping device as a whole. The flexible display panel 2 will be described in detail below.

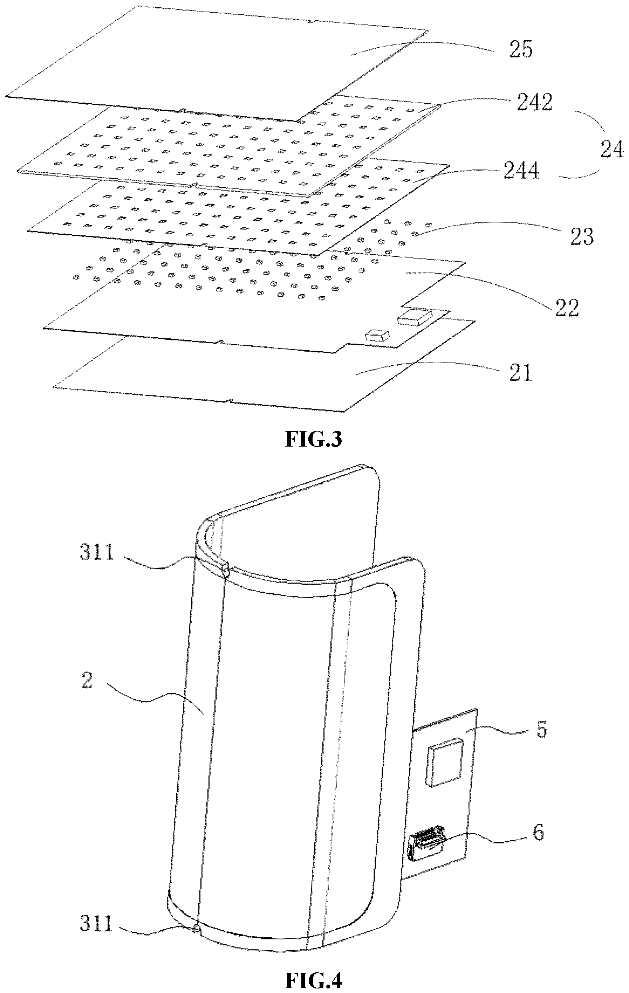

Refer to Figure 3, in some embodiments, the flexible display panel 2 comprises a flexible mounting layer 21, a flexible substrate layer 22, a plurality of light-emitting devices 23 and a flexible light-shielding layer 24.

The flexible mounting layer 21 is fixed on at least two end surfaces of the housing 1. For example, one side of the flexible mounting layer 21 is fixed to at least two end surfaces of the housing 1, and the other side of the flexible mounting layer 21 is designed to fix the flexible substrate layer 22. In some embodiments, the flexible mounting layer 21 is a first adhesive tape, which can be pasted on the surface of the housing 1 and the surface of the flexible substrate layer 22 to fix the housing 1 and the flexible substrate layer 22. In this embodiment, the first adhesive tape may be double-sided adhesive tape, or may be a viscous and flexible material such as cured glue, so the first adhesive tape can be bent to a certain extent.

One side of the flexible substrate layer 22 is fixed to the flexible mounting layer 21, and a circuit is arranged on the other side of the flexible substrate layer 22. In some embodiments, the flexible substrate layer 22 may be a flexible printed circuit (FPC), which uses polyimide or polyester film as a base material. This flexible printed circuit (FPC) has high reliability, excellent flexibility, high wiring density, and is light, thin, and has good bendability. Therefore, the flexible printed circuit (FPC) can also be bent to a certain extent, and a circuit is configured on it to implement a preset circuit function, for example, to drive the light-emitting device 23 to emit light.

Each light - emitting device 23 is fixed on the other side of the flexible substrate layer 22 and is electrically connected to the circuit on the flexible substrate layer 22. In some embodiments, the light - emitting device 23 is designed to emit light when the circuit inputs a current to it, and the magnitude of the current can control the brightness of the light - emitting device 23. In some embodiments, the light - emitting device 23 is an LED chip, and the LED chip is fixed to the flexible substrate layer 22 through die - bonding. The die - bonding is an existing process - that is, through colloid (for LED chips, it is generally conductive glue or insulating glue) the LED chip is bonded to a designated area of the bracket to form a thermal path or an electrical path, thereby ensuring stable electrical connection and physical support between the LED chip and the flexible substrate layer 22.

In some embodiments, when at least part of the flexible display panel 2 is bent in a first direction and then fixed to at least two end surfaces of the housing 1, if the first direction is a vertical direction, then the length direction of the LED chip on the bent portion of the flexible display panel 2 is the horizontal direction. If the first direction is the horizontal direction, the length direction of the LED chip on the flexible display panel 2 in the bent portion is the vertical direction. In this embodiment, when the flexible display panel 2 is bent, its bent portion will generate a certain amount of stress, and the stress is basically consistent with the direction of bending. For example, if the left and right ends of the flexible display panel 2 are bent in a horizontal direction, then corresponding stress will be generated in the bent portion in the middle of its left and right ends. Otherwise, if its upper and lower ends are bent in the vertical direction, corresponding stress will be generated in the bent portion in the middle of its upper and lower ends. Since the LED chip is not flexible, the LED chip on the flexible display panel 2 in the bent portion needs to withstand the stress, which may damage the LED chip. To address this issue, in order to reduce the impact of stress caused by bending on the LED chip, the length direction of the LED chip on the bent portion of the flexible display panel 2 is perpendicular to the direction in which the flexible display panel 2 is bent, thereby reducing the impact of stress on the LED chip. For example, when the left and right ends of the flexible display panel 2 are bent horizontally, the length direction of the LED chips on the bent part is vertical.

The flexible light-shielding layer 24 is fixed on the other side of the flexible substrate layer 22 and covers the plurality of light-emitting devices 23. In some embodiments, the flexible light-shielding layer 24 covers the plurality of light-emitting devices 23 so that only part of the light can be transmitted, thereby displaying preset content. For example, the flexible light-shielding layer 24 is provided with a plurality of light-transmitting holes of a predetermined shape. The holes are designed to display corresponding preset shape information when the light-emitting device 23 emits light, and the combination of the preset shape information displayed between each light-transmitting hole is designed to represent the device status of the electronic vaping device. For example, the light-transmitting holes can be in the shape of numbers, so that the corresponding numbers are displayed when the light-emitting device 23 emits light. The light-transmitting holes can also be in various shapes such as text shapes, letter shapes, dot shapes, line shapes, etc., so that a combination of various shape information is designed to represent the device status of the electronic vaping device. In some embodiments, the device status may comprise working mode, monitoring parameters, alarm status, charging status, etc.

In some embodiments, the flexible light-shielding layer 24 includes a second adhesive tape 244 and a flexible light-shielding sheet 242. Wherein, the second adhesive tape 244 is pasted on the other side of the flexible substrate layer 22, and the flexible light-shielding sheet 242 is pasted on the second adhesive tape. In this embodiment, the second adhesive tape 244 can be made of the same material as the first adhesive tape, and the flexible light-shielding sheet 242 can be made of silicone material that can shield light, so the flexible light-shielding layer 24 can also be bent to a certain extent.

In some embodiments, in order to implement colorful emission of the flexible display panel 2, the flexible display panel 2 further comprises a flexible colorful film layer 25 fixed to the flexible light - shielding layer 24. The flexible colorful film layer 25 has a colorful area corresponding to the light - transmitting holes of the flexible light - shielding layer 24. The colorful area is designed to display a preset color when the light - emitting device 23 emits light through it. In some embodiments, the flexible colorful film layer 25 filters the unwanted color in the white light based on the principle of three primary colors, thereby emitting light of the desired color. After that, light with the desired color is gained. The light - emitting device 23 is designed to emit white light. For example, a blue LED chip emits blue light, and the blue light then passes through phosphor to emit white light. In some embodiments, the flexible colorful film layer 25 includes a PET (polyethylene terephthalate) material layer configured with a film for filtering light and forming a colorful area. In some embodiments, the flexible colorful film layer 25 also includes a diffusion film fixed on the PET material layer. The diffusion film is designed to adjust the light into a uniform surface light source to achieve an optical diffusion effect, thereby achieving better display effects. Therefore, the flexible colorful film layer 25 can also be bent to a certain extent.

In some embodiments, in order to implement colorful lighting of the flexible display panel 2, the light-emitting device 23 can also be configured to emit at least one selected from the group consisting of red light, blue light, and green light; and the control component is designed to control the light-emitting device 23 based on at least one selected from the group consisting of red light, blue light, and green light to emit light of a preset color. For example, the light-emitting device 23 may include a blue LED chip, a red LED chip, and a green LED chip, so that based on the three primary colors principle, light of a preset color may be emitted.

In the above embodiments, the flexible display panel 2 is implemented based on the flexible mounting layer 21, the flexible substrate layer 22, the plurality of light - emitting devices 23 and the flexible light - shielding layer 24, so that it can be arranged on multiple end surfaces. The flexible display panel 2 is assembled into a layered structure with various flexible layers combined. Compared with the rigid structure of PCB and digital tubes, the layered structure formed by combining various flexible layers greatly reduces the overall thickness. Therefore, the flexible display panel 2 occupies less space, which facilitates the portability of the electronic vaping device.

Refer to Figure 4, in some embodiments, if at least part of the area of the flexible display panel 2 is bent and then fixed to the housing 1, the installation process of the flexible display panel 2 includes the following steps.

The plurality of light-emitting devices 23 are mounted on the flexible substrate layer 22, and at least part of the area where the flexible substrate layer 22 needs to be bent is bent to put the flexible substrate layer 22 in a bent state. Then the flexible light-shielding layer 24 is bent into a state corresponding to that of the bent flexible substrate layer 22, and then the bent flexible light-shielding layer 24 is bonded to one side of the bent flexible substrate layer 22. After that, the other side of the bent flexible substrate layer 22 is fixed to at least two end surfaces of the housing 1 through the flexible mounting layer 21.

In the above embodiment, if each layer of the flexible display panel 2 is installed prior to bending at least part of its area, the bonding between the layers will loosen due to the bending stress during the bending process. As a result, the display effect of the display panel 2 may be severely affected, and the flexible display panel 2 may even be damaged. Regarding this, during the installation process, after completing the installation of the light - emitting device, the flexible display panel 2 is bent before the flexible light - shielding layer, so that the flexible light - shielding layer in the bent state is fixed to the flexible substrate layer in the bent state. This can avoid various problems caused by bending stress.

The above are some descriptions of the flexible display panel 2. The sealing structure of the flexible display panel 2 and the housing 1 will be described in detail below.

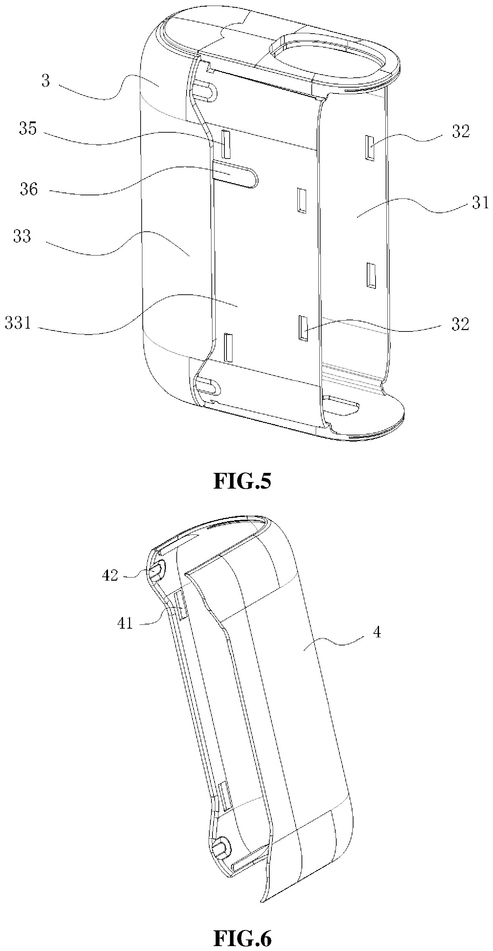

Refer to Figure 5 and Figure 6, in some embodiments, the electronic vaping device also comprises a first housing 3 and a second housing 4. The first housing 3 and the second housing 4 are designed to jointly wrap the entire side surface of the housing 1, wherein the first housing 3 is mainly designed to wrap and cover the flexible display panel 2 to form isolation and protection for the flexible display panel 2. The second housing 4 assists in wrapping and fixing the first housing 3.

In some embodiments, the first housing 3 can be a transparent or semi-transparent housing, that is, the first housing 3, which has a transparent or semi-transparent structure as a whole, completely covers the flexible display panel 2, and the user can view the display content of flexible display panel 2 through the first housing 3. The first housing 3 can be an integrated structure, so that the first housing 3 has a more stable structure and can also improve the sealing effect of the flexible display panel 2 wrapped by the first housing 3.

In some embodiments, the first housing 3 may be provided with a transparent window, and other parts of the first housing 3 are non-transparent housings. The transparent window is made of transparent or semi-transparent material, and the transparent window is located outside the flexible display panel 2. The transparent window provided in the first housing 3, thus, also allows the user to view the flexible display panel 2.

In some embodiments, the first housing 3 may be a structure with an opening 31. The size of the opening 31 of the first housing 3 corresponds to the size of the second side surface of the housing 1. The opening 31 of the first housing 3 is larger than the size of the second side surface of the housing 1, so that the housing 1 can be inserted into the first housing 3 from the opening 31. After the housing 1 is inserted into the first housing 3, the first housing 3 covers the first side surface 11, the second side surface, the third side surface 13, the top surface 15 and the bottom surface of the housing 1. The first housing 3 wraps and covers at least part of the first side surface 11 and the third side surface 13, and the first housing 3 covers all of the second side surface, the top surface 15 and the bottom surface. The fourth side surface 14 of the housing 1 is not covered by the first housing 3, and the entire flexible display panel 2 is covered by the first housing 3.

In some embodiments, the first snap fit portion 17 may be provided on the side surface of the housing 1. For example, a plurality of first snap fit portions 17 may be provided on the first side surface 11 and the third side surface 13 of the housing 1. A first snap fit portion 17 can be a protruding buckle or other structure with a snap fit function. The inner surface of the first housing 3 is provided with a second snap fit portion 32. The number and position of the second snap fit portion 32 correspond to the first snap fit portion 17. The second snap fit portion 32 may be a slot or a bayonet. The connection between the first snap fit portion 17 and the second snap fit portion 32 can fix the first housing 3 to the outside of the housing 1.

In some embodiments, the second snap fit portions 32 can be distributed close to the opening 31, so that the snap fit position between the first housing 3 and the housing 1 is close to the opening 31, which can avoid the opening 31 being unfixed and improve the stability when the first housing 3 is installed on the housing 1.

In some embodiments, the first snap fit portion 17 can be a one-way buckle. The one-way buckle has opposite guide surfaces and limiting surfaces. The guide surface is inclined to the side surface where the housing 1 is located, the limiting surfaces are perpendicular to the side surface where the housing 1 is located. When the first housing 3 is being connected to the housing 1, the second snap fit portion 32 can slide along the guide surface of the one-way buckle until the one-way buckle snaps into the bayonet or slot, The limiting surface of the one-way buckle can prevent the first housing 3 and the housing 1 from moving in the disassembly direction. The provision of the one-way buckle can improve the stability of the fixed connection between the first housing 3 and the housing 1 and prevent the first housing 3 from accidentally moving out.

In some embodiments, the connection between the first snap fit portion 17 and the second snap fit portion 32 not only plays a fixing role, but also plays a positioning role, allowing the first housing 3 to be accurately installed on the side surface of the housing 1.

In some embodiments, the first housing 3 is also provided with a relief opening for the mouthpiece 151 and a relief opening for the air - inlet. The mouthpiece 151 can be inserted into the housing 1 through the relief opening of the first housing 3.

In some embodiments, the first snap fit portion 17 can also be a slot or a bayonet, and the second snap fit portion 32 can be a protruding buckle, and the connection between the first snap fit portion 17 and the second snap fit portion 32 can also be implemented to fix the first housing 3 to the outside of the housing 1.

In some embodiments, the first housing 3 can also be directly and fixedly connected to the housing 1 via screw connection or bonding.

In some embodiments, the second housing 4 may have a C-shaped structure. The second housing 4 is wrapped around the outside of the fourth side surface 14 of the housing 1, and both sides of the second housing 4 are also wrapped around the first housing 3 respectively. The first housing 3 has a fifth side surface 33 and a sixth side surface. The fifth side surface 33 is located outside the first side surface 11 of the housing 1. The sixth side surface is located outside the third side surface 13 of the housing 1. The second housing 4 is connected to and partially covers the fifth side surface 33 and the sixth side surface of the first housing 3. The second housing 4 covers the fourth side surface 14 of the housing 1 that is not covered by the first housing 3, or the second housing 4 covers the fourth side surface 14 of the housing 1 that is not covered by the first housing 3 and part of the third side surface. The second housing 4 wraps the edge area of the opening 31 of the first housing 3. The first housing 3 and the second housing 4 form a cabbage-like inner and outer laminated structure, which can completely wrap the side surface of the housing 1, and the first housing 3 and the second housing 4 partially overlap without forming a gap.

The fifth side surface 33 and the sixth side surface of the second housing 4 are respectively provided with a plurality of third snap fit portions 35, On the inner side of the second housing 4, there are fourth snap fit portions 41, the quantity and positions of which correspond one-to-one to those of the third snap fit portions 35. one of the third snap fit portion 35 and the fourth snap fit portion 41 may be a clasp, and the other may be a snap fit slot or a bayonet. The connection between the third snap fit portion 35 and the fourth snap fit portion 41 can fix the second housing 4 to the outside of the first housing 3.

Similarly, the fourth snap fit portion 41 can be arranged close to the edge of the second housing 4, so that the connection between the third snap fit portion 35 and the fourth snap fit portion 41 can fix the edge position of the second housing 4, improving the stability of the second housing 4's fixation and preventing the edge of the second housing 4 from turning out or warping.

The buckle in the third snap fit portion 35 and the fourth snap fit portion 41 can also be a one - way buckle, which further improves the stability of the installation and fixation of the second housing 4 and prevents the second housing 4 from detaching.

In the above embodiments, since the first housing 3 and the second housing 4 are provided on the outside of the housing 1, the first housing 3 wraps and covers the flexible display panel 2 installed on the housing 1 and part of the side surface of the housing 1. Then, the second housing 4 wraps the side surface of the housing 1 that is not covered by the first housing 3 and the edge area of the first housing 3. The first housing 3 and the second housing 4 form an inner - outer laminated wrapping manner, which can wrap all the side surfaces of the housing 1 without any gap space. This is beneficial for enhancing the aesthetic appeal of the electronic vaping device. It can also prevent external media such as liquids from entering the flexible display panel 2 or other components through the gaps in the housing 1, which helps to extend the service life of the flexible display panel 2 and other components.

In some embodiments, the first snap fit portion 17 arranged on the first side surface 11 and those on the third side surface 13 of the housing 1 are asymmetrically staggered. This arrangement makes it impossible to install the first housing 3 on the housing 1 after the first housing 3 is turned over, serving as a fool - proof measure to prevent the first housing 3 from being installed in the wrong direction.

In some embodiments, the third snap fit portions 35 arranged on the fifth side surface 33 and the sixth side surface of the first housing 3 are asymmetric and staggered. The fourth snap fit portions 41 on both sides of the second housing 4 are correspondingly staggered, which can also serve as a fool - proof measure to prevent the second housing 4 from being installed in the wrong direction.

In some embodiments, one or more parallel first guiding connection portions 36 are respectively provided on the first sunken surface 331 and the second sunken surface of the first housing 3. The first guiding connection portions 36 can be in the structure of guiding strips or guiding grooves, and the length direction of the first guiding connection portions 36 is parallel to the top surface 15 and the bottom surface. On the inner side of the second housing 4, there are second guiding connection portions 42 corresponding to the first guiding connection portions 36 one by one. The second guiding connection portions 42 are guiding grooves that fit the guiding strips, or guiding strips that fit the guiding grooves. The second housing 4 can be inserted onto the first housing 3 along the length direction of the first guiding connection portions 36 and wrap the housing 1.

The guiding connection between the first guiding connection portion 36 and the second guiding connection portion 42 can play a role in guiding and positioning the installation of the second housing 4, enabling the second housing 4 to be aligned with the first housing 3. Moreover, the first guiding connection portion 36 and the second guiding connection portion 42 can also function as a limit, preventing the second housing 4 from moving in a direction perpendicular to the first guiding connection portion 36 and the second guiding connection portion 42.

The first guiding connection portions 36 are provided on both the first sunken surface 331 and the second sunken surface of the first housing 3, which can improve the stability of the guided installation of the second housing 4.

In some embodiments, the first guiding connection portion 36 is provided on either the first sunken surface 331 or the second sunken surface, and the inner side of the second housing 4 is provided with the second guiding connection portion 42 corresponding to the first guiding connection portion 36. In this way, the guided installation, positioning, and fixation of the second housing 4 can also be achieved.

The above are some descriptions about the sealing of the flexible display panel 2 and the housing 1. The following will provide a specific description of the driving control of the flexible display panel 2.

Refer to Figure 2, in some embodiments, the electronic vaping device further includes a screen control panel 5 and a connector 6. The flexible display panel 2, the screen control panel 5, and the connector 6 are connected as one to form a modular structure. The flexible display panel 2, the screen control panel 5, and the connector 6 are installed together on at least two end surfaces of the housing 1.

In some embodiments, the screen control panel 5 is arranged side by side with the flexible display panel 2. The screen control panel 5 and the flexible display panel 2 can be laid flat on the side surface of the housing 1. This arrangement facilitates the installation of the flexible display panel 2 and the screen control panel 5.The connector 6 can be provided on the screen control panel 5, and it can be placed on the front side of the screen control panel 5. The back side of the screen control panel 5 faces the housing 1, while the front side faces away from the housing 1. Having the connector 6 on the front side of the screen control panel 5 makes it convenient to connect the control component to the connector 6.

In some embodiments, the screen control panel 5 can be formed by the extension of the flexible substrate layer in the flexible display panel 2. For example, the screen control panel 5 can be obtained by configuring a display panel control circuit on the extended flexible substrate layer. In some other embodiments, the screen control panel 5 can also be arranged on the back side of the flexible display panel 2. A part of the screen control panel 5 extends out from one side of the flexible display panel 2, and the connector 6 is installed on the extended part of the screen control panel 5. This structure can also form an integrated modular structure, which is beneficial to the installation of the flexible display panel 2.

In some embodiments, the side surface of the housing 1 is provided with a first accommodating groove 18 and a second accommodating groove 19 arranged side by side. The first accommodating groove 18 can extend to multiple side surfaces of the housing 1, and its distribution corresponds to that of the flexible display panel 2. As shown in Figure 4, the first accommodating groove 18 is distributed on the first, second, and third side surfaces of the housing 1, forming a C - shaped curved distribution. The second accommodating groove 19 is arranged on the first side surface of the housing 1, and there is a communicating gap between the first accommodating groove 18 and the second accommodating groove 19.

The shape and size of the first accommodating groove 18 are adapted to those of the flexible display panel 2. The flexible display panel 2 is installed in the first accommodating groove 18, and the four edges of the flexible display panel 2 are close to or in contact with the four side walls of the first accommodating groove 18. This helps to reduce the gap between the first accommodating groove 18 and the flexible display panel 2, enables the first accommodating groove 18 to limit the position of the flexible display panel 2, and enhances the aesthetic appearance.

The shape and size of the second accommodating groove 19 are adapted to those of the screen control panel 5. The screen control panel 5 is installed in the second accommodating groove 19. Similarly, the four edges of the screen control panel 5 are close to or in contact with the four side walls of the second accommodating groove 19 to reduce the gap between the second accommodating groove 19 and the screen control panel 5 and to enable the second accommodating groove 19 to limit the position of the screen control panel 5.

The depth of the first accommodating groove 18 is equal to or approximately equal to the thickness of the flexible display panel 2, and the depth of the second accommodating groove 19 is equal to or approximately equal to the thickness of the screen control panel 5. In this way, after the flexible display panel 2 and the screen control panel 5 are respectively installed in the first accommodating groove 18 and the second accommodating groove 19, the front surfaces of the flexible display panel 2 and the screen control panel 5 can be flush with the side surface of the housing 1, which is more aesthetically pleasing. It can also play a role in hiding and protecting the flexible display panel 2 and the screen control panel 5.

In some embodiments, one or both of the flexible display panel 2 and the screen control panel 5 can also be directly installed on the side surface of the housing 1, and in this way, the installation of the flexible display panel 2 and the screen control panel 5 can also be achieved.

In some embodiments, the screen control panel 5 can also be fixed inside the second accommodating groove 19 by using adhesive such as double - sided foam 3M tape. Since the screen control panel 5 has a flat - plate structure, it can also be fixedly connected to the housing 1 through methods such as snap fit or screw connection.

In some embodiments, the screen control panel 5 is electrically connected to both the flexible display panel 2 and the connector 6 respectively. The screen control panel 5 is used to receive the control signals from the control component and control the display content of the flexible display panel 2 based on these control signals. The connector 6 is used for making an electrical connection with the control component.

The connector 6 can be a connection structure such as a socket with a jack. The circuit component can be plugged into the connector 6 through a flexible flat cable. The housing 1 can be provided with an avoidance opening 12. One end of the flexible flat cable is connected to the circuit board 4, and the other end passes through the avoidance opening 12 of the housing 1 to be plugged into the connector 6.

In some embodiments, the second accommodating groove 19 can be arranged at a position close to the bottom surface. The screen control panel 5 is located at a position closer to the lower end on one side of the flexible display panel 2. The control component can be installed on the bottom surface of the housing 1, and the avoidance opening 12 can be arranged close to the second accommodating groove 19. In this way, after one end of the flexible flat cable passes through the avoidance opening 12, it only needs to extend a short distance to be plugged into the connector 6. This can reduce the length of the flexible flat cable, lower the cost, and improve the stability of transmission.

Refer to Figure 2 and Figure 4, in some embodiments, a first positioning portion 111 can be provided inside the first accommodating groove 18, and the flexible display panel 2 can be provided with a second positioning portion 311 corresponding to the first positioning portion 111. The first positioning portion 111 can be one or more protrusions located on the side wall of the edge of the first accommodating groove 18, and the flexible display panel 2 has an equal number of recesses at corresponding positions. When the flexible display panel 2 is installed in the first accommodating groove 18, the protrusions of the first positioning portion 111 are snapped into the recesses of the second positioning portion 311. With this arrangement, the positioning of the flexible display panel 2 in the direction of its bending length can be achieved, and the flexible display panel 2 can be prevented from moving along the direction of its bending length.

Wherein, the first positioning portion 111 is preferably arranged inside the second side surface of the housing 1 where the first accommodating groove 18 is located. Moreover, one first positioning portion 111 is respectively arranged at the upper and lower ends of the first accommodating groove 18. Correspondingly, a second positioning portion 311 is respectively arranged at the upper and lower ends of the middle position of the flexible screen. In this way, the first positioning portion 111 can position the middle position of the flexible screen. When the flexible screen is bonded to the housing 1, it can be first bonded to the second side surface of the housing 1 after being positioned at the middle, and then bent to be bonded to the first and third side surfaces of the housing 1. This can make the flexible screen more perfectly aligned with the first accommodating groove 18 and avoid mis-installation of the flexible screen.

In the above - mentioned embodiments, integrating the flexible display panel 2, the screen control panel 5, and the connector 6 facilitates installation and electrical connection. Meanwhile, the integrated structure can be more easily incorporated into other products and adapted to the circuit boards of other products, which improves the expandability and compatibility of the flexible display panel 2.

The above are some descriptions about the driving control of the flexible display panel 2. The following will provide a specific description of the control of the display content of the flexible display panel 2.

In some embodiments, the flexible display panel 2 has at least two display areas, which are correspondingly distributed on at least two end surfaces of the housing 1. The control component is used to control each display area to show content. Hereinafter, the flexible display panel 2 having a main display area, a first sub - display area, and a second sub - display area will be taken as an example for illustration. For example, the main display area is distributed on the second or fourth side surface of the housing 1, while the first sub - display area and the second sub - display area are respectively distributed on the first and third side surfaces of the housing 1.

In this embodiment, the electronic vaping device can obtain control instructions through the interaction component for the purpose of controlling the electronic vaping device. Wherein, when the control component obtains a mode control instruction based on the interaction component, the control component responds to the mode control instruction and controls the vaping component to enter the corresponding working mode, enabling the vaping component to vape the vaping substrate with the working parameters corresponding to this working mode. For example, when the vaping component has two heating wires, the vaping component can have a single-heating mode and a double-heating mode. When the user operates through the interaction component to enter the single-heating mode, the control component controls one of the heating wires of the vaping component to work. When entering the double-heating mode, the control component controls both heating wires of the vaping component to work.

The control component obtains the mode display information used to represent the working mode of the vaping component based on this working mode, as well as the first auxiliary display information and/or the second auxiliary display information associated with the mode display information. In some embodiments, the mode display information can be in the form of patterns, numbers, text, letters, symbols, etc. Different working modes can correspond to different types of mode display information, for example, using patterns and symbols respectively for display, or they can correspond to the same type of mode display information, such as using patterns for separate display.

In some embodiments, one or both of the first auxiliary display information and the second auxiliary display information can be obtained based on one or both of the mode display information and the corresponding working mode of the vaping component. It only needs to ensure that the first auxiliary display information and the second auxiliary display information are associated with the mode display information. For example, the association can be achieved through the corresponding display content and the mode display information.

In some embodiments, the first auxiliary display information and the second auxiliary display information can also be in the form of patterns, numbers, text, letters, symbols, etc.

In some embodiments, the first auxiliary display information includes dynamic prompt display information corresponding to the mode display information. This dynamic prompt display information can be information that flashes at a certain frequency. Alternatively, the dynamic prompt display information may consist of multiple local display information items, and these multiple local display information items are displayed sequentially in chronological order or cyclically. In this embodiment, the dynamic prompt display information is defined in contrast to static display information. Therefore, any display information that is not static and unchanging can be regarded as dynamic prompt display information, and different display information can be designed according to requirements.

In some embodiments, the first auxiliary display information may also include static prompt display information corresponding to the mode display information.

In some embodiments, the mode display information can include static display information and may also include dynamic display information. When the first auxiliary display information and the second auxiliary display information include dynamic prompt display information, the mode display information can be static display information, so as to clearly distinguish the display mode of the mode display information from that of the first and second auxiliary display information.

In some embodiments, the second auxiliary display information also includes dynamic prompt display information corresponding to the mode display information. Here, the second auxiliary display information may have the same content as the first auxiliary display information, so no further details will be provided here. The second auxiliary display information may also have different or partially different content from the first auxiliary display information, and it can be adjusted as needed.

In some embodiments, the first auxiliary display information includes multiple first prompt display information items that gradually get closer to the main display area over time. For example, these multiple first prompt display information items are distributed in a way that they gradually approach the main display area from a distance. As time passes, the first prompt display information farther away from the main display area is shown first, and then the ones closer to the main display area are gradually displayed. In some embodiments, the first auxiliary display information may also include multiple first prompt display information items that gradually move away from the main display area over time. That is, as time changes, the first prompt display information closer to the main display area is shown first, and then the ones farther away from the main display area are gradually displayed. In some embodiments, the first auxiliary display information may include both multiple first prompt display information items that gradually move away from the main display area and multiple first prompt display information items that gradually get closer to the main display area. In this embodiment, when the multiple first prompt display information items gradually approach or move away from the main display area, it could mean that adjacent first prompt display information items successively get closer to or farther away from the main display area. It could also mean that among the first prompt display information items, those separated by one or more other first prompt display information items successively get closer to or farther away from the main display area.

In some embodiments, the first prompt display information can be dot patterns or line patterns. Therefore, the combination of dot patterns and line patterns among multiple first prompt display information items can form a pipeline that gradually approaches or moves away from the main display area. In some embodiments, the second auxiliary display information also includes multiple second prompt display information items that gradually get closer to the main display area over time, and/or multiple second prompt display information items that gradually move away from the main display area over time. The second auxiliary display information can be the same as the first auxiliary display information, and thus no further details will be provided here. It can also be different or partially different from the first auxiliary display information.

The control component controls the main display area to display the mode display information. If the first auxiliary display information is obtained, it also controls the first auxiliary display area to display the first auxiliary display information. If the second auxiliary display information is obtained, it also controls the second auxiliary display area to display the second auxiliary display information. If both the first auxiliary display information and the second auxiliary display information are obtained, it controls the first auxiliary display area to display the first auxiliary display information and the second auxiliary display area to display the second auxiliary display information.

In the above embodiment, the flexible display panel 2 has a main display area, a first auxiliary display area, and a second auxiliary display area. The main display area is used to display the mode display information, while the auxiliary display areas are used to display the auxiliary display information associated with the mode display information. With multiple display areas on different end - surfaces of the main display area and the auxiliary display areas, users can more easily aware of the mode display information. As a result, users can quickly obtain the mode display information based on these multiple display areas, thereby enhancing the user experience.

In the above embodiment, the main display area is located between the first auxiliary display area and the second auxiliary display area, so that the mode display information can be prominently displayed. Meanwhile, the auxiliary display areas can show the dynamic prompt display information corresponding to the mode display information. Moreover, the auxiliary display areas can display multiple prompt display information items that gradually approach or move away from the main display area. With the prompt information in the auxiliary display areas, the mode display information can be further highlighted, enabling users to better aware of the mode display information. Users can also quickly obtain the mode display information based on the prompt information in the auxiliary display areas, thus enhancing the user experience.

In some embodiments, when the control component controls the vaping component to enter the first working mode, it obtains the first mode display information representing the first working mode. When the control component controls the vaping component to enter the second working mode, it obtains the second mode display information representing the second working mode. The display content and display positions of the first mode display information and the second mode display information are not completely the same. For example, the display content may be the same, but the display position changes; the display position may be the same, but the display content changes; or both the display content and the display position may change.

The control component also obtains the first auxiliary display information of the first mode that is associated with the first mode display information, and obtains the second auxiliary display information of the second mode that is associated with the second mode display information.

In some embodiments, the control component also obtains the second auxiliary display information of the first mode that is associated with the first mode display information, and obtains the first auxiliary display information of the second mode that is associated with the second mode display information.

In some embodiments, the control component also obtains the first auxiliary display information of the first mode and the second auxiliary display information of the first mode that are associated with the first mode display information, and obtains the second auxiliary display information of the second mode or the first auxiliary display information of the second mode that is associated with the second mode display information.

In some embodiments, the control component also obtains the first auxiliary display information of the first mode or the second auxiliary display information of the first mode that is associated with the first mode display information, and obtains the second auxiliary display information of the second mode and the first auxiliary display information of the second mode that are associated with the second mode display information.

In the above embodiments, when the vaping component enters different working modes, not only can the auxiliary display information corresponding to different working modes be different to be associated with the corresponding mode display information, but also the display areas where the auxiliary display information corresponding to different working modes is displayed can be different to be associated with the corresponding mode display information.

The above is some explanation of one kind of display content control for the flexible display panel 2. The following will explain another kind of display content control.

The control component is used to obtain the current device status of the electronic vaping device. For example, it obtains the device status of the electronic vaping device through the status monitoring component. The device status of the electronic vaping device includes at least one of the working mode, alarm status, charging status, and monitored parameters. The working mode refers to the working mode of the vaping component, the charging status includes the charging state and the fully charged state, the monitored parameters include the remaining battery level and the remaining vaping substrate, the alarm status includes the low battery alarm and the low vaping substrate alarm. In some embodiments, the status monitoring component can obtain the monitored parameters and the charging status based on various detection circuits or sensors, can obtain the working mode based on the interaction component described in the above embodiments, and can obtain the alarm status based on the data processing processor.

The control component controls one of the display areas to display the first display information, which is used to represent the current device status. The first display information can be in the form of patterns, numbers, text, letters, symbols, etc. Different device statuses can correspond to different types of first display information. For example, patterns and symbols can be used for display respectively, or they can correspond to the same type of first display information, such as using patterns for separate display.

If the current device status changes, the control component controls at least one other display area to display the second display information, which is different from the first display information and is used to represent that the current device status has changed. The control component also controls one of the above-mentioned display areas to adjust from displaying the first display information to displaying the third display information, and the third display information is used to represent the changed device status. In some embodiments, the second display information can also be in the form of patterns, numbers, text, letters, symbols, etc.

In some embodiments, when the at least one other display area includes at least two display areas, the control component can control these at least two display areas to alternately display the second display information. Alternatively, it can control these at least two display areas to display the second display information simultaneously. Or, it can control one of these at least two display areas to dynamically display the second display information and the other to statically display the second display information.

In some embodiments, the second display information includes dynamic prompt display information corresponding to the first display information. This dynamic prompt display information can adopt the dynamic prompt display information corresponding to the first auxiliary display information in the above - mentioned embodiments, and thus no further details will be provided here.

In some embodiments, the second display information includes multiple prompt display information items that gradually approach and are displayed towards one of the display areas over time, and/or multiple prompt display information items that gradually move away from and are displayed away from one of the display areas over time. These multiple prompt display information items can be the same as the multiple prompt display information items corresponding to the first auxiliary display information in the above-mentioned embodiments, so no further details will be provided here.

In the above embodiments, the device status is displayed based on one of the display areas. When the device status changes, corresponding prompt information is displayed based on the other display areas. This enables users to better aware of the changed device status based on the prompt information from the other display areas on different end surfaces, and thus quickly obtain the device status of the electronic vaping device.

The above is some explanation of another kind of display content control for the flexible display panel 2. The following will explain yet another kind of display content control.

The control component is used to select at least two display areas and configure the information types corresponding to the information displayed in each display area. In some embodiments, the selected display areas can be all the display areas of the flexible display panel 2, or they can be some of the display areas. In some embodiments, the information displayed in the display area can correspond to one or more information types, or it may not correspond to any information type.

The control component obtains the current device status of the electronic vaping device and determines the information type corresponding to this current device status. In some embodiments, the device status of the electronic vaping device includes at least one of the working mode, alarm status, charging status, and monitored parameters, and the working mode, alarm status, charging status, and monitored parameters respectively correspond to one information type.

The control component controls the corresponding display area to display the first display information according to the information type of the current device status. The first display information is used to represent the current device status. For example, if the current device status includes the working mode, then the display area corresponding to the working mode displays the display information representing the working mode. Wherein, when the working mode is the first working mode, the display information representing the first working mode is displayed.

The control component controls at least one other display area to display the second display information, which is associated with the first display information. In this embodiment, the corresponding second display information can be obtained based on the first display information, so that the second display information is associated with the first display information. Therefore, each device state has its corresponding first display information and second display information.

In some embodiments, the second display information includes dynamic prompt display information corresponding to the first display information. This dynamic prompt display information can adopt the dynamic prompt display information corresponding to the first auxiliary display information in the above - mentioned embodiments, and thus no further details will be provided here.

In some embodiments, the second display information includes multiple prompt display information items that gradually approach and are displayed towards the corresponding display area over time, and/or multiple prompt display information items that gradually move away from and are displayed away from the corresponding display area over time. These multiple prompt display information items can be the same as the multiple prompt display information items corresponding to the first auxiliary display information in the above - mentioned embodiments, so no further details will be provided here.

In some embodiments, when the at least one other display area consists of at least two display areas, the control component can control these at least two display areas to alternately display the second display information. Alternatively, it can control these at least two display areas to display the second display information simultaneously. Or, it can control one of these at least two display areas to dynamically display the second display information while the other statically displays it.

In the above embodiments, the information displayed in the selected display areas is classified by information type, enabling each display area to show the corresponding information type, When the device status is obtained, the device status is displayed in the display area corresponding to the information type. This division based on information types makes it convenient for users to obtain the device status corresponding to each information type. Meanwhile, by displaying prompt information associated with the device status in other display areas, users can more easily focus on the relevant device status based on the information displayed in multiple display areas on different end - faces. As a result, they can quickly obtain the device status information of the electronic vaping device, thereby enhancing the user experience.

In some embodiments, the information displayed in each display area corresponds to at least one information type, and the information types corresponding to different display areas are all different. This enables different types of display information to be respectively displayed based on multiple display areas, avoiding the situation where the display information corresponding to the device status of various information types is crowded in one display area, which may stop users obtaining the device status information of the electronic vaping device quickly.

Those ordinary skilled person in the art can understand that all or part of the functions of the various methods in the above embodiments can be implemented in a hardware manner or through a computer program. When all or part of the functions in the above embodiments are implemented through a computer program, the program can be stored in a computer-readable storage medium. The storage medium may include: read-only memory, random access memory, magnetic disks, optical discs, hard disks, etc., and the above functions can be achieved by a computer executing the program. For example, if the program is stored in the memory of a device, when the processor executes the program in the memory, all or part of the above functions can be achieved. In addition, when all or part of the functions in the above embodiments are implemented through a computer program, the program can also be stored in storage media such as a server, another computer, a magnetic disk, an optical disc, a flash drive, or a mobile hard disk. It can be downloaded or copied and saved in the memory of a local device, or the system of the local device can be updated to a new version. When the processor executes the program in the memory, all or part of the functions in the above embodiments can be achieved.

The above has illustrated the present application based on specific examples, which is only for helping to understand the present application and is not intended to limit the present application. For ordinary skilled person in the art, based on the ideas of the present application, several simple deductions, deformations, or replacements can also be made.

## Claims

1. An electronic vaping device comprising:
a housing, inside which a reservoir is configured to store vaping substrate and an air outlet channel is formed in the housing;
a mouthpiece configured on the housing and connected with the air outlet channel;
a vaping component, configured in the housing, the vaping component is designed to vape the vaping substrate to generate aerosol which passes through the air outlet channel to the mouthpiece;
a flexible display panel configured on the housing for displaying visual information;
a control component respectively connected to the flexible display panel and the vaping component;
the housing comprises a top surface, a bottom surface, and a plurality of side surfaces that connect the top surface and the bottom surface, the plurality of side surfaces are connected with each other in sequence, the mouthpiece is arranged at a preset position of the top surface;
the plurality of side surfaces comprise gripping area and non-gripping area;
the gripping area is designed for gripping the housing, and the distribution position of the gripping area has a preset positional relationship with the preset position of the mouthpiece, so that when at least part of the gripping area is being gripped by a user, the mouthpiece approaches and/or faces the user;
the flexible display panel is configured on one or more selected from the group consisting of the non-gripping area, the top surface, and the bottom surface,
the flexible display panel is distributed on at least two selected from the group consisting of the top surface, bottom surface, and the plurality of side surfaces.

2. The electronic vaping device of claim 1, wherein the plurality of side surfaces comprise a first side surface, a second side surface, a third side surface and a fourth side surface that are connected in sequence, and the widths of the second side surface and the fourth side surface are both smaller than the width of the first side surface and the third side surface;
the gripping area is an area included in the second side surface, and at least part of the area included in the first side surface that is connected to the second side surface, and/or an area included in at least part of the third side surface that is connected to the second side surface, the other areas are non-gripping areas;
or,
the gripping area is an area included in the fourth side surface, and at least part of the area included in the first side surface that is connected to the fourth side surface, and/or an area included in at least part of the third side surface that is connected to the fourth side surface, the other areas are non-gripping areas.

3. The electronic vaping device of claim 2, wherein the preset position is a position close to the second side surface or the fourth side surface.

4. An electronic vaping device comprising:
a housing, inside which a reservoir is configured to store vaping substrate and an air outlet channel is formed in the housing
a vaping component, configured in the housing, the vaping component is designed to vape the vaping substrate to generate aerosol which passes through the air outlet channel to the outside of the housing;
a flexible display panel configured on the housing for displaying visual information;
a control component respectively connected to the flexible display panel and the vaping component;
the housing comprises a plurality of end surfaces that connect with each other in sequence, the plurality of end surfaces comprise gripping area and non-gripping area, the gripping area is designed for gripping the housing, the flexible display panel is configured in the non-gripping area and distributed on at least two end surfaces of the plurality of end surfaces.

5. The electronic vaping device of claim 4 comprising:
a mouthpiece configured at a preset position on one of the end surfaces of the housing, the mouthpiece is connected to the air outlet channel; the preset position of the mouthpiece has a preset positional relationship with the distribution position of the gripping area.

6. The electronic vaping device of claim 5, wherein the preset positional relationship make the gripping area closer to the mouthpiece than the non-gripping area, or make the gripping area further away from the mouthpiece than the non-gripping area.

7. The electronic vaping device of claim 4, wherein the flexible display panel comprises a flexible mounting layer, a flexible substrate layer, a plurality of light-emitting devices and a flexible light-shielding layer;
the flexible mounting layer is fixed to at least two end surfaces of the housing;
one side of the flexible substrate layer is fixed to the flexible mounting layer, and the other side of the flexible substrate layer is configured with circuit;
each of the light-emitting devices is fixed on the other side of the flexible substrate layer and connected to the circuit;
the flexible light-shielding layer is fixed on the other side of the flexible substrate layer and covers the plurality of light-emitting devices;
each of the light-emitting devices is configured to emit light when the circuit inputs a driving current to it, the flexible light-shielding layer is provided with a plurality of light-transmitting holes with preset shapes, the light-transmitting holes with preset shapes are configured to display corresponding preset shape information when light generated by light-emitting devices is transmitted through it, and the combination of the preset shape information displayed between each of the light-transmitting holes is designed to represent the device status of the electronic vaping device.

8. The electronic vaping device of claim 7, wherein the light-emitting device is an LED chip, and the LED chip is fixed to the flexible substrate layer by die bonding.

9. The electronic vaping device of claim 8, wherein at least part of the flexible display panel is bent in a first direction and then fixed to the housing; if the first direction is a vertical direction, then the length direction of the LED chip on the flexible display panel in the bent portion is the horizontal direction, if the first direction is the horizontal direction, then the length direction of the LED chip on the flexible display panel in the bent portion is the vertical direction.

10. The electronic vaping device of claim 7, wherein if at least part of the flexible display panel is bent and fixed to the housing, then the installation process of the flexible display panel comprises:
mount a plurality of light-emitting devices to the flexible substrate layer, and bend at least part of the flexible substrate layer to obtain a flexible substrate layer in a bent state;
bent the flexible light-shielding layer correspondingly to obtain a flexible light-shielding layer in a bent state, and the flexible light-shielding layer in a bent state is bonded to and fixed on one side of the flexible substrate layer in a bent state;
fix the other surface of the bent flexible substrate layer to the at least two end surfaces of the housing by the flexible mounting layer.

11. The electronic vaping device of claim 4, wherein the flexible display panel has at least two display areas, correspondingly distributed on the at least two end surfaces, and the control component is designed for:
select at least two of the display areas and configure the information type corresponding to the information displayed in each of the display areas;
obtain the current device status of the electronic vaping device, determine the information type corresponding to the current device status, and control the corresponding display area to display the first display information according to the information type of the current device status, the first display information configured to present the current device status;
control at least one other display area to display second display information, wherein the second display information is associated with the first display information.

12. The electronic vaping device of claim 4, comprises a first housing and a second housing, the first housing is disposed outside the housing, the first housing wraps a portion of the plurality end surfaces of the housing and covers the flexible display panel; The area where the first housing covers the flexible display panel is a transparent or semi-transparent structure;
the second housing is disposed on the outside of the housing, the second housing wraps other portion of the plurality end surfaces of the housing, and the second housing wraps the edge region of the first housing.

13. The electronic vaping device of claim 12, wherein the first housing is a housing structure with an opening on one side, and the first housing can insert into the housing through the opening, so that the first housing wraps a portion region of the housing's plurality of end surfaces, other portion region of the housing's plurality of end surfaces is exposed to the opening; the second housing is a C-shaped housing structure, and the second housing wraps the other portion region of the housing's plurality of end surfaces.

14. The electronic vaping device of claim 4 comprises:
a screen control panel and a connector;
wherein the flexible display panel is integrally connected to the screen control panel and the connector, the screen control panel is electrically connected to the flexible display panel and the connector respectively, the connector is configured to connect to the control component.

15. An electronic vaping device, comprising:
a housing, inside which a reservoir is configured to store vaping substrate and an air outlet channel is formed in the housing
a mouthpiece configured on the housing and connected with the air outlet channel;
a vaping component, configured in the housing, the vaping component is designed to vape the vaping substrate to generate aerosol which passes through the air outlet channel to the mouthpiece;
a flexible display panel configured on the housing for displaying visual information;
a control component respectively connected to the flexible display panel and the vaping component;
the housing comprises a top surface, a bottom surface, and a plurality of side surfaces that connect the top surface and the bottom surface, the plurality of side surfaces are connected with each other in sequence, the mouthpiece is arranged at a preset position of the top surface, the flexible display panel is annularly configured on the plurality of side surfaces.
